(19) Europäisches Patentamt
European Patent Office
Office européen des brevets

(11) Publication number: **0 087 858**
**A1**

(12) # EUROPEAN PATENT APPLICATION

(21) Application number: 83300578.8

(22) Date of filing: 04.02.83

(51) Int. Cl.³: C 07 D 311/22
C 07 D 307/83, A 61 K 31/35
A 61 K 31/34

(30) Priority: 05.02.82 JP 17971/82

(43) Date of publication of application:
07.09.83 Bulletin 83/36

(84) Designated Contracting States:
AT BE CH DE FR GB IT LI LU NL SE

(71) Applicant: NIPPON ZOKI PHARMACEUTICAL CO. LTD.
10, 2-chome Hirano-machi Higashi-ku
Osaka(JP)

(72) Inventor: Okamoto, Kaoru
Nippon Zoki Pharm. Co Ltd 10, 2-chome, Hiranomachi
Higashi-ku Osaka(JP)

(72) Inventor: Hamada, Masaaki
Nippon Zoki Pharm. Co Ltd 10, 2-chome, Hiranomachi
Higashi-ku Osaka(JP)

(72) Inventor: Kurosaki, Teikichi
Nippon Zoki Pharm. Co Ltd 10, 2-chome, Hiranomachi
Higashi-ku Osaka(JP)

(74) Representative: Crampton, Keith John Allen et al,
D YOUNG & CO 10 Staple Inn
London WC1V 7RD(GB)

(54) **New chromanone compounds and pharmaceutical compositions containing them.**

(57) Chromanone compounds represented by the general formula (I);

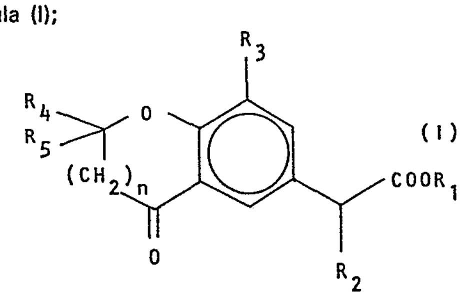

(I)

in which each of $R_1$, $R_2$, $R_4$ and $R_5$ is a hydrogen atom or a lower alkyl group, $R_3$ is a hydrogen or halogen atom, n is 0 or 1 and their pharmaceutical salts, are novel compounds. They have been found to possess analgesic, antipyretic and antiinflammatory properties. They may be made up into various pharmaceutical compositions, which are suitable for treating diseases mainifesting pain, fever and/or inflamma-tion.

EP 0 087 858 A1

Croydon Printing Company Ltd.

# NEW CHROMANONE COMPOUNDS AND PHARMACEUTICAL COMPOSITIONS CONTAINING THEM

The present invention is concerned with chromanone compounds and pharmaceutical compositions containing them.

Although compounds such as salicylates, anthranilic acid derivatives, phenylacetic acid derivatives, indolylacetic acid derivatives and pyrazolones have been used conventionally as drugs having antipyretic, analgesic and antiinflammatory actions, they have given rise to troubles due to side effects such as gastro-intestinal, hepatorenal and hematological disorders. Alleviation of these adverse effects has been attempted by improving dosage forms and by chemical modification, or more recently by other methods, e.g. the use of pro-drugs.

The present invention results from investigations for developing drugs comparable or superior to conventional non-steroid drugs in effectiveness, and without the above-mentioned side effects. As a result of these investigations, some new chromanone compounds were discovered, and certain of these compounds were found to have antiiflammatory and antipyretic-analgesic actions, as well as low toxicity, thus showing pharmaceutical utility.

The present invention provides compounds represented by the following general formula (I);

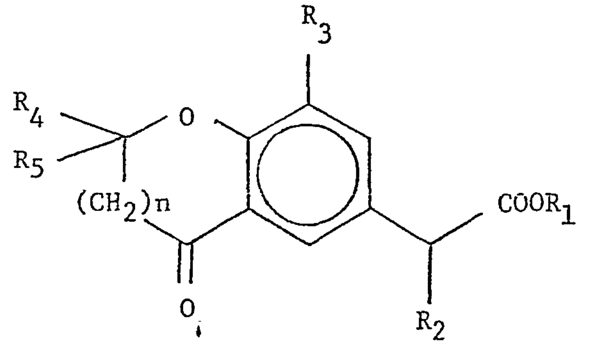

(I)

or are pharmaceutically acceptable salts of such compounds. In Formula (I), each of $R_1$, $R_2$, $R_4$ and $R_5$ is a hydrogen atom or lower alkyl group, or $R_4$ and $R_5$, together with the carbon atom to which they are attached, form

a ring having 3 to 6 members, $R_3$ is a hydrogen or halogen atom, n is 0 or 1. The invention also provides pharmaceutical compositions containing one or more such compounds as active ingredient.

Values of $R_1$ and $R_2$, in addition to hydrogen, include straight or branched lower alkyl groups preferably having 1 to 4 carbon atoms such as methyl, ethyl, propyl, isopropyl, butyl, iso-butyl, sec-butyl and t-butyl. The lower alkyl groups may contain up to 5 or even 6 carbon atoms.

Values or $R_3$ include hydrogen, fluorine, chlorine, bromine and iodine.

Values of $R_4$ and $R_5$ include those mentioned above for $R_1$ and $R_2$. In addition, $R_4$ and $R_5$ may optionally together form 3 to 6 membered ring (cyclopropyl, cyclobutyl, cyclopentyl, cyclohexyl).

When optical isomers exist in the compounds of the present invention, the invention includes dl-, l- and d-isomers.

The preferred pharmaceutically acceptable salts of the compounds of formula (I) are those with alkali metals such as sodium, potassium and lithium, with alkaline-earth metals such as calcium and magnesium, with other metals such as aluminium, with ammonium, and with organic amines such as trimethylamine, triethylamine, pyrrolidine, piperidine, morpholine, pyridine and picoline.

In accordance with the present invention, the new compounds are prepared as follows, the variables being as defined above:

A compound represented by the general formula (II);

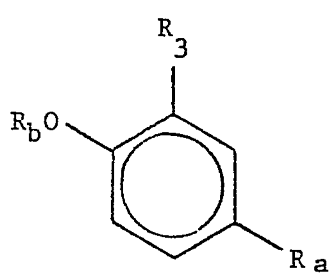

(II)

(in which Ra is a hydrogen atom, a lower alkyl group or $-CHR_2-COOR_1$ and Rb is a hydrogen atom or a lower alkyl group) is reacted with a compound represented by the general formula (III) or an active derivative thereof;

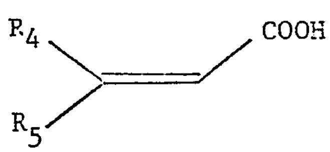

(III)

to give a compound represented by the general formula (IV)

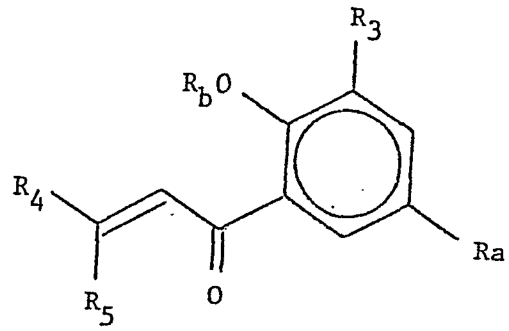

(IV)

and compound (IV) is converted to a compound represented by the general formula (I) by ring closure with acid.

To carry out the reaction, the compound represented by the general formula (IV) may be obtained by Friedel-Crafts reaction with anhydrous aluminium chloride in an inert solvent such as carbon disulphide, chloroform, carbon tetrachloride or methylene chloride.

Then the compound (IV) is heated with strong acid such as polyphosphoric acid, concentrated hydrochloric acid or concentrated sulphuric acid at an appropriate temperature to yield by ring closure the intended compound. It furthermore is not always necessary to separate the compound of formula (IV). The compounds represented by the general formulae (II) and (III) and the above-mentioned acid catalyst may be reacted together simultaneously to give a compound of the present invention in one step.

Alternatively, a compound of formula (II) above is reacted with a compound represented by the general formula (V) in the same way as with the first process

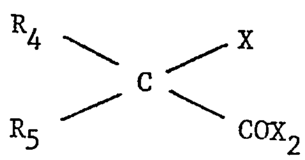

(V)

in which each of $X_1$ and $X_2$ indicates a halogen atom to give a compound represented by the general formula (VI);

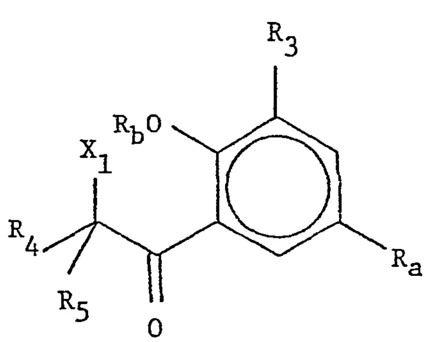

(VI)

which is then converted to a compound of the present invention by ring closure. To carry out the ring closure reaction, the compound (VI) is allowed to stand at room temperature or at an appropriate temperature in the presence of an alkali metal carbonate such as potassium carbonate, optionally with reaction-accelerating agent such as potassium iodide, in an inert solvent such as dimethylformamide.

In another method, a compound represented by the general formula (II) in which Rb = H is reacted with β-propiolactone in the presence of base such as sodium hydroxide with heating at an appropriate temperature to give a compound represented by the general formula (VII);

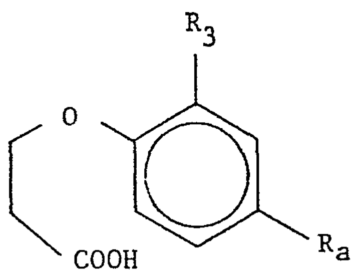

(VII)

and the compound (VII) is then converted by ring closure in the same way as in the first process to give a compound represented by the general formula (I) in which $R_4 = R_5 = H$.

Yet another method involves reacting a compound represented by the general formula (II), in which Rb = H with a compound represented by the general formula (VIII);

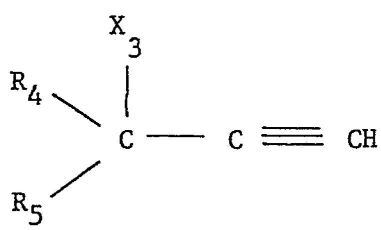

(VIII)

in which $X_3$ indicates a halogen atom, followed by ring closure with heating and reduction to give a compound represented by the general formula (IX); (ref. Japanese patent application No. Sho-56-126912)

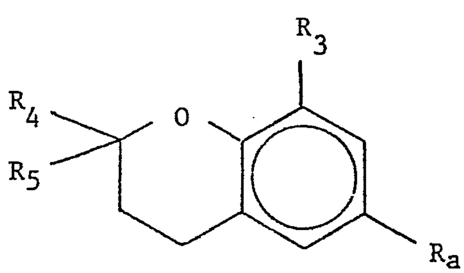

(IX)

The resultant compound is then oxidized to give a compound of the present invention. Potassium permanganate, lead tetraacetate or chromic anhydride may be used as oxidizing reagent.

In all the above-mentioned reactions, when a compound represented by the general formula (II) in which Ra is a hydrogen atom or a lower alkyl group is used, Ra can be treated in the usual way to give a compound of the present invention either during the synthesis or after.

For example, when Ra is a hydrogen atom, the compound (II) is acylated by Friedel-Crafts reaction and then treatment of the acyl group with thallium (III) nitrate, which results in oxidative rearrangement to give a group $-CHR_2-COOR_1$ according to the method of Taylor [ Journal of Synthetic Organic Chemistry, Japan, 36, 875 (1978) ].

When Ra is a lower alkyl group, the compound (II) may be halogenated with a halogenating reagent such as N-bromosuccinimide, and then converted to a nitrile with cyanating reagent such as sodium cyanide, potassium cyanide, followed by hydrolysis in the usual way to give the compound of the present invention.

In accordance with the above-mentioned processes, the compound of the present invention is obtained, in the form of free carboxylic acid or ester. Furthermore, it may be converted to another compound of the present invention by esterification or hydrolysis in the usual way.

For example, free carboxylic acids of the present invention may be rected with an alcohol corresponding to the intended $R_1$ in the presence of an acid such as sulphuric acid, gaseous hydrogen chloride, hydrochloric acid, p-toluenesulphonic acid, camphorsulphonic acid or thionyl chloride to give ester derivatives of the present invention. The hydrolysis may be carried out in water, an organic solvent such as a lower alcohol, or mixture of them, in the presence of a base such as sodium hydroxide, potassium hydroxide, sodium carbonate, potassium carbonate, sodium bicarbonate or potassium bicarbonate, or an acid catalyst such as hydrochloric acid, sulphuric acid, acetic acid or p-toluenesulphonic acid. In the case of benzyl ester derivatives, the ester residue may be removed by reduction, e.g. with hydrogen over a palladium-on-carbon catalyst.

The compounds of the present invention also may be converted to their salts, if desired, in the usual ways.

The compounds of the present invention can be purified by usual methods such as distillation, chromatography and recrystallization, and the products can be identified by such methods as elemental analysis, melting point and IR, NMR, UV and mass spectra.

The examples, which follow, illustrate the preparations of compounds of the present invention. NMR was measured using TMS (tetramethylsilane) as an internal standard and expressed in the delta value; parts and percentages are by weight, the pressures 1, 1.5 and 2 mmHg correspond to 133, 200 and 266 Pa respectively.

Example 1.

(1) 108 g of p-cresol was added to 10% aqueous solution of sodium hydroxide with heating at 100°C. 72 g of β-propiolactone was further added dropwise to the mixture with stirring. After stirring for 15 minuts, the reaction mixture was cooled at 30°C and acidified with concentrated hydrochloric acid to give 27 g of crystalline 3-(4-methylphenoxy)propionic acid.

(2) 200 g of polyphosphoric acid was added to the resultant crystals, then the reaction mixture was stirred at 95°C for 20 minutes. Under cooling with ice, water was added to the mixture followed by extraction with ethyl acetate and distillation using a Claisen flask to give 19.5 g of oily matter (b.p. 91-93 °C/1.5 mm/Hg) of 6-methyl-4-chromanone.

(3) The resultant oily matter was reacted with 24.6 g of N-bromosuccinimide under a nitrogen atmosphere in 100 ml of carbon tetrachloride and refluxed under ultraviolet irradiation. After cooling, the precipitate was filtered off and the filtrate was washed with a 10% aqueous solution of sodium hydroxide and saturated aqueous solution of sodium chloride, and dried over anhydrous sodium sulphate. Then solvent was distilled off. The residue was recrystallized from mixtures of n-hexane and ethyl acetate to give 13 g of needle-like crystals of 6-bromomethyl-4-chromanone.

(4) The resultant crystals were dissolved in 35 ml of dimethylformamide. The solution was added dropwise at 7-8°C to a mixture of 3 g of sodium cyanide and 65 ml of dimethylformamide and stirred at room temperature for 2 hours. The reaction mixture was poured into ice-water, and the resultant crystals were dried and recrystallized from n-hexane-ethyl acetate to give 6 g of (4-chromanon-6-yl)-acetonitrile.

(5) 4.7 g of the product of step (4) was added to a mixture of 5 ml of water, 5 ml of acetic acid, and 4 ml of concentrated sulphuric acid, and refluxed for 2 hours. The reaction mixture was poured into ice-water followed by extraction with chloroform. The organic layer was washed with water and a saturated aqueous solution of sodium chloride, and dried over anhydrous sodium sulphate. Solvent was distilled off. The resultant crude product was recrystallized from methylene chloride to give 3.6 g of needle-like crystals of (4-chromanon-6-yl) acetic acid (Compound 1)

m.p. 108 - 110°C

IR(KBr) 3500-2100, 1721, 1652, 1615, 1570, 1495, 1316, 1223, 1178, 1030, 875, 780, 650 $cm^{-1}$

NMR(Acetone-$d_6$) $\delta$ = 2.74(t,2H), 3.61(s,2H), 4.52(t,2H), 6.88(d,1H), 7.41(dd,1H), 7.69(d,1H), 8.35(bs,1H)

Example 2.

(1) A mixture of 25 g of p-cresol, 28 g of 3-methylcrotonic acid and 200 g of polyphosphoric acid was stirred at 80-85°C for 1 hour. Ice-water was added to the reaction mixture which was then extracted with ether. The ethereal layer was washed with water, a saturated aqueous solution of sodium bicarbonate and a saturated aqueous solution of sodium chloride. Solvent was distilled off and the residue was distilled using a Claisen flask to give 35 g of oily matter (b.p. 115-117°C/2 mmHg) of 2,2,6-trimethyl-4-chromanone.

(2) In the same way as in example 1(3)(4)(5), white needle-like crystals of (2,2-dimethyl-4-chromanon-6-yl) acetic acid (Compound 2) were obtained as a result of the conversion of the 6-methyl group.

m.p. 139 - 141°C

IR(KBr) 3500-2100, 2980, 1720, 1650, 1618, 1572, 1492, 1305, 1250, 1200, 1160, 922, 890, 830, 750, 575 $cm^{-1}$

NMR(Acetone-$d_6$) $\delta$ = 1.42(s,6H), 2.71(s,2H), 3.60(s,2H), 6.83(d,1H), 7.41(dd,1H), 7.68(d,1H), 8.68(bs,1H)

Example 3.

(1) A mixture of 10 g of methyl 2-(8-chloro-2,2-dimethylchroman-6-yl)propionate, 200 ml of acetic acid and 5.7 g of chromic anhydride was stirred at room temperature for 2.5 hours. Water was added to the mixture which was extracted with ether. The ethereal layer was washed with water, a saturated aqueous solution of sodium bicarbonate and a saturated

aqueous solution of sodium chloride, and then dried over anhydrous sodium sulphate. The resultant crude product was purified by column chromatography on silica gel (n-hexane-ethyl acetate) to give 5.5 g of methyl 2-(8-chloro-2,2-dimethyl-4-chromanon-6-yl)propionate as an oil.

(2) 20 ml of concentrated hydrochloric acid was added to the product and the reaction mixture was refluxed for 3 hours. Water was added to the mixture, which was then extracted with ether. The ethereal layer was washed with water and a saturated aqueous solution of sodium chloride and dried over anhydrous sodium sulphate. Solvent was distilled off. Then the residue was purified by column chromatography (benzene-ethyl acetate) and recrystallized from mixtures of n-hexane and ethyl acetate to give 3.9 g of white crystals of 2-(8-chloro-2,2-dimethyl-4-chromanon-6-yl)propionic acid (Compound 3).

m.p. 94 - 96°C

IR(KBr) 3500-2200, 2970, 1705, 1683, 1601, 1465, 1295, 1268, 1170, 930, 888, 845, 565 $cm^{-1}$

NMR(Acetone-$d_6$) $\delta$ = 1.47(d,3H), 1.48(s,6H), 2.81(s,2H), 3.77(q,1H), 7.59(d,1H), 7.66(d,1H), 9.3(bs,1H)

Example 4.

(1) 9.0 g of methyl 2-(4-methoxyphenyl)propionate, which had been dissolved in 30 ml of carbon disulphide, was added dropwise to a mixture of 15.0 g of anhydrous aluminium chloride and 60 ml of carbon disulfide. 5.6 ml of 3-methylcrotonic acid chloride was added dropwise to the reaction mixture under reflux. Reaction was continued for 30 minutes. Water was added to the reaction mixture, which was extracted with ether. The ethereal layer was washed with water, a saturated aqueous solution of sodium bicarbonate and a saturated aqueous solution of sodium chloride, and then dried over anhydrous sodium sulphate. Solvent was distilled off. Then the residue was purified by column chromatography on silica gel (benzene-ethyl acetate) to give 6.0 g of methyl 2-[4-hydroxy-3-(3-methylcrotonoyl)phenyl]propionate as an oil.

(2) The product was added to a mixture of 60 ml of water, 60 ml of acetic acid and 6 ml of concentrated sulphuric acid and the reaction mixture was refluxed for 5 hours. In the same way as in example 3(2), 4.0 g of white crystals of 2-(2,2-dimethyl-4-chromanon-6-yl)propionic acid (Compound 4) were obtained.

m.p. 97 - 99°C

IR(KBr) 3600-2200, 2970, 1700, 1690, 1602, 1562, 1481, 1300, 1259, 1215, 1168, 924, 910 $cm^{-1}$

NMR($CDCl_3$) $\delta$ = 1.45(s,6H), 1.51(d,3H), 2.70(s,2H), 3.75(q,1H), 6.85(d,1H), 7.41(dd,1H), 7.75(d,1H), 10.98(bs,1H)

Example 5.

(1) A mixture of 22 g of 3-(2-chlorophenoxy)propionic acid, 250 ml of dichloroethane, 9 ml of methanol and 1 ml of concentrated hydrochloric acid was refluxed for 14 hours. After cooling, the mixture was washed with a saturated aqueous solution of sodium bicarbonate and a saturated aqueous solution of sodium chloride. Solvent was distilled off and the residue was distilled using a Claisen flask to give 23 g of methyl 3-(2-chlorophenoxy)propionate (b.p. 115-120°C/1 mmHg).

(2) 20 ml of carbon disulphide containing the resultant ester was added to a mixture of 30 g of anhydrous aluminium chloride and 60 ml of carbon disulphide under cooling with ice. 14 ml of propionic anhydride was further added dropwise to the reaction mixture under reflux. After 30 minutes, the mixture was poured into water and extracted with ethyl acetate. The organic layer was washed in the same way as in (1) and dried. Solvent was then removed. The crude product was recrystallized from mixtures of n-hexane and ethyl acetate to give 16 g of white crystals of 3-chloro-4-methoxycarbonylethoxypropiophenone (m.p. 71-73°C).

3) 15.5 g of the product and 150 ml of carbon tetrachloride was added to a reagent prepared by stirring for several minutes and concentrating to dryness a mixture of 76 ml of methyl orthoformate, 61 ml of methanol, 30 g of thallium (I) nitrate and 68 g of silica gel. Then the reaction mixture was refluxed for 4 hours. After cooling, the precipitate was filtered off and then filtrate was extracted with ethyl acetate.The organic layer was washed with water, a saturated aqueous solution of sodium bicarbonate and a saturated aqueous solution of sodium chloride and dried over anhydrous sodium sulphate. Solvent was distilled off and the residue was purified by column chromatography on silica gel (benzene-ethyl acetate) to give 15 g of oily matter of methyl (2-(3-chloro-4-methoxycarbonylethoxyphenyl)propionate.

(4) In the same way as in example 3(2), hydrolysis was carried out to give 8 g of white crystals of 2-(3-chloro-4-carboxyethoxyphenyl)propionic acid (m.p. 133-135°C).

(5) 80 ml of concentrated sulphuric acid was added to the resultant crystals. The mixture was stirred at room temperature for 4.5 hours and then poured into water, followed by extraction with ethyl acetate. The organic layer was washed with water and dried. Solvent was distilled off. Then the residue was recrystallized from mixtures of n-hexane and ethyl acetate to give 6.3 g of white needle-like crystals of 2-(8-chloro-4-chromanon-6-yl)propionic acid (Compound 5).

m.p. 139 - 142°C

IR(KBr) 3500-2000, 1715, 1648, 1597, 1482, 1239, 1179, 1010, 890, 868, 832, 721, 642 $cm^{-1}$

NMR(Acetone-$d_6$) $\delta$ = 1.48(d,3H), 2.83(t,2H), 3.73(q,1H), 4.65(t,2H), 7.56(d,1H), 7.70(d,1H), 8.45(bs,1H)

Example 6.

(1) In the same way as in example 4(1), methyl 2-(4-methoxyphenyl)propionate was reacted with 2-bromo-iso-butyrylbromide to give methyl 2-[3-(2-bromo-isobutyryl)-4-hydroxyphenyl] propionate as an oil.

(2) 4.0 g of potassium carbonate, 5.0 g of potassium iodide and 60 ml of dimethylformamide were added to 6.5 g of the resultant oil under a nitrogen atmosphere. The reaction mixture was stirred at 80°C for 30 minutes, and then poured into ice-water, followed by extraction with ether. The ethereal layer was washed with water and a saturated aqueous solution of sodium chloride, and dried over anhydrous sodium sulphate. Solvent was distilled off. The residue was purified by column chromatography on silica gel (n-hexane-ethyl acetate) to give 3.2 g of methyl 2-(2,2-dimethyl-2,3-dihydrobenzofuran-3-one-5-yl)propionate as an oil.

(3) 5 g of the oil was dissolved in 50 ml of methanol. 5 ml of a 50% aqueous solution of potassium hydroxide was added to the solution which was refluxed for 1 hour. Purification of the product was carried out in the usual way to give 4.5 g of crystals of 2-(2,2-dimethyl-2,3-dihydrobenzofuran-3-one-5-yl)propionic acid (Compound 6).

m.p. 83 - 85°C

IR(KBr) 3600-2200, 2970, 1720, 1700, 1615, 1590, 1483, 1265, 1112, 939, 830, 801, 742, 592 $cm^{-1}$

NMR($CDCl_3$) $\delta$ = 1.45(s,6H), 1.51(d,3H), 3.76(q,1H), 6.71(d,1H), 7.59(dd,1H), 7.61(d,1h), 10.77(bs,1H)

The compounds of the present invention have new structures different from conventional non-steroid antiinflammatory compounds. Compounds of the present invention have remarkably potent antiinflammatory, analgesic and antipyretic effectss, as well as low toxicity.

The following description services to illustrate animal studies.

(1) Acute toxicity

Groups of 10 male dd mice weighing some 18 g were orally or intraperitoneally administered the examined drugs and the number of death up to 72 hours later was recorded.

An example of results is shown in Table 1: the drugs are those numbered correspondingly above. Administration is oral (p.o.) or intraperitoneal (i.p.)

Table 1

| drug | dosage (mg/kg) | mortality (%) |
|---|---|---|
| Compound 2 | 600 (p.o.) | 0 |
| Compound 3 | 400 (p.o.) | 0 |
| Compound 4 | 300 (p.o.) | 0 |
| | 200 (i.p.) | 0 |
| Compound 5 | 400 (p.o.) | 0 |
| | 800 (p.o.) | 0 |
| Compound 6 | 300 (p.o.) | 0 |
| | 200 (i.p.) | 0 |
| Phenylbutazone | 400 (p.o.) | 30 |
| Aminopyrine | 600 (p.o.) | 20 |

(2) Inhibition of plantaroedema induced by carrageenin

60 minutes after the drugs had been orally administered to groups of rats, 0.1 ml of 1% carrageenin solution was injected

subcutaneously into the sole. The rate of increase in volume of the hind food compared with volume before carrageenin treatment was regarded as oedema rate.

An example of results is shown in Table 2.

Table 2

| drug | dosage (mg/kg) | oedema rate (%) |
|---|---|---|
| Compound 2 | 100 | 46 |
| Compound 3 | 100 | 48 |
| Compound 4 | 50 | 48 |
| Compound 6 | 50 | 34 |
| | 100 | 53 |
| Phenylbutazone | 50 | 33 |
| | 100 | 50 |
| Ibuprofen | 100 | 54 |

Furthermore, sections of the stomach and intestinal tracts of the rats, after the above-mentioned observations had been made, revealed mucosal disorder or ulcer formation in the phenylbutazone-administered group, while no abnormality was found in any animals in the groups that received the compounds of the present invention.

(3) Analgesic effects by a modified Haffner method

Groups of 10 dd mice were orally administered the examined drugs and 30 minutes later 2 mg/kg of morphine hydrochloride, the threshold dose, was injected subcutaneously. At 15, 30, 45 and 60 minutes thereafter, the root of mouse's tail was squeezed with a Kocher's forceps. The number of animals that did not exhibit pseudopain reactions was recorded and the determination with the largest number of responding animals among the four determinations was used for evaluation.

An example of results is shown in Table 3.

Table 3

| drug | analgesic effect (%) | |
|---|---|---|
| | 50 mg/kg | 100 mg/kg |
| Compound 1 | 15 | 30 |
| Compound 3 | 20 | 25 |
| Compound 5 | 20 | 45 |
| Compound 6 | 20 | 40 |
| Aminopyrine | 20 | 35 |

(4) Analgesic effects by acetic acid writhing test

30 minutes after the examined drugs were given orally, 0.1 ml/10 g of 0.6% acetic acid solution was intraperitoneally administered to groups of 10 male dd mice weighing some 18 g. The number of stretching was calculated during an observation period of 15 to 20 minutes after acetic acid treatment. The average inhibition rate of stretching among 8 animals was regarded as the inhibition rate of the drug, excluding 2 animals showing maximum and minimum number of stretching.

An example of results is shown in Table 4.

Table 4

| drug | analgesic effect (%) | |
|---|---|---|
| | 50 mg/kg | 100 mg/kg |
| Compound 1 | 31 | 45 |
| Compound 2 | 13 | 62 |
| Compound 3 | 27 | 59 |
| Aminopyrine | 17 | 57 |

(5) Gastric irritation

8 hours after the examined drugs were orally administered to groups of rats, degree of gastric irritation/ulceration produced by the drug is compared with that produced by aspirin (150 mg/kg) or indomethacin (40 mg/kg). Percentage rate of production of gastric irritation, compared with the control drug, (that produced by aspirin or indomethacin was taken as 100%), was regarded as the gastric irritation rate.

An example of results is shown in Table 5.

Table 5

| drug | dosage (mg/kg) | gastric irritation rate(%) |
|---|---|---|
| Compound 4 | 50 | 8 |
| Compound 6 | 100 | 25 |

As the above-mentioned animal experiments clearly indicate, compounds of the present invention have excellent anti-inflammatory and analgesic action. Therefore, these compounds are therapeutically available antiinflammatory, analgesic and antipyretic agents against various inflammations such as those in rheumatic diseases, arthritis, and other inflammations having a variety of reddening, fever, swelling and pain, against painful diseases and manifestations such as acute and chronic pains, neuralgia, trauma, lumbago, and pains accompanied by inflammation, and against a variety of symptoms involving fever. Furthermore, compounds of the present invention have no gastric irritation action, which is a contraindication for many conventional drugs.

The compounds of the present invention can be made into pharmaceuticals by combination with appropriate medical carriers or diluents, and dosage forms in solid, semisolid, liquid or gaseous form can be prepared in the usual way for oral or non-oral administration.

On preparation of pharmaceutical dosage forms, the compounds of the present invention may be used in the form of their pharmaceutically acceptable salts, and they also can be used alone or in

appropriate association with one another, as well as in association with other pharmaceutically active components.

When the compounds are applied orally, they may be used alone or combined with appropriate fillers to make tablets or capsules, e.g. with coventional bases such as lactose, mannitol, corn starch and potato starch, with binders such as crystalline cellulose, cellulose derivatives, acacia, corn starch and gelatin, with disintegrators such as corn starch, potato starch and sodium carboxymethylcellulose and with lubricants such as talc and magnesium stearate. They also can be combined with ointment bases such as vaseline, paraffin, plastibase, simple ointment, hydrophilic ointment, hydrophilic petrolatum and hydrophilic plastibase to make ointments. They may also be mixed thoroughly with a variety of bases such as emulsifying bases or water-soluble bases to give suppositories.

As regards injectable forms, the compounds of the present invention can be administered as solutions or suspensions in aqueous solvents or non-aqueous solvents such as vegetable oils, synthetic aliphatic acid glycerides, esters of higher aliphatic acids and propylene glycol.

When utilized as inhalation or aerosol preparations, the compounds of the present invention in the form of liquid or minute powder can be filled up in an aerosol container, with gas or liquid spraying agents and with conventional adjuvants such as humidifying of dispersing agents added, if necessary. The compounds of the present invention also may be applied as pharmaceuticals for non-pressurized preparations such as in a nebulizer or an atomizer.

Poultices can be prepared by mixing the compounds with mentha oil, concentrated glycerin, kaolin or other suitable additives.

The desirable dose of the compounds of the present invention varies with the subject, method and period of administration, but generally it is recommended to administer orally 10 to 3,000 mg of these compounds daily to an adult to obtain the desired effects. One to several units of the unit preparation containing the compounds of the present invention in appropriate amount may be administered.

As for non-oral administration (e.g. for injectable forms), daily doses of one tenth to one third of the above oral doses are desirable.

Some prescriptions of the pharmaceutical compositions are shown below as examples which contain the compounds of the present invention as active ingredients.

Prescription example 1. (Tablet)

| components | content of a tablet (mg) |
|---|---|
| an invented compound | 100 |
| lactose | 130 |
| corn starch | 40 |
| magnesium stearate | 10 |
| total | 280 mg |

Prescription example 2. (Capsule)

| components | content of a capsule (mg) |
|---|---|
| an invented compound | 50 |
| lactose | 250 |
| total | 300 mg |

Prescription example 3. (injection)

| components | content of an ampoule (mg) |
|---|---|
| an invented compound | 10 |
| sodium chloride | proper amount |
| distilled water for injection | proper amount |
| total | 1 ml |

Prescription example 4. (ointment)

| components | weight (g) |
|---|---|
| an invented compound | 1 |
| emulsified wax | 30 |
| white petrolatum | 50 |
| liquid paraffin | 20 |
| total | 101 g |

Prescription example 5. (suppository)

| components | content of a suppository (mg) |
|---|---|
| an invented compound | 20 |
| cacao butter | 1980 |
| total | 2000 mg |

## CLAIMS

1. Chromanone compounds represented by the general formula (I);

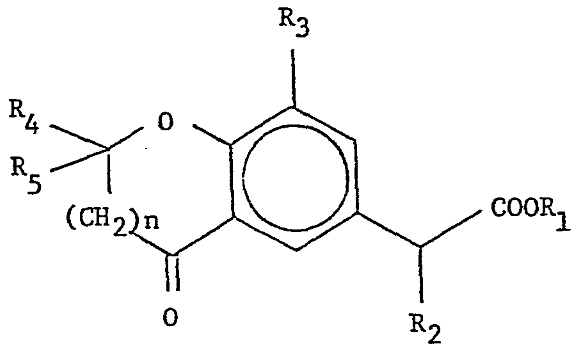

(I)

in which each of $R_1$, $R_2$, $R_4$ and $R_5$ is a hydrogen atom or a lower alkyl group, $R_3$ is a hydrogen or halogen atom, n is 0 or 1 and compounds that are their pharmaceutically acceptable salts.

2. Compounds according to Claim 1 in which n is 1.

3. Compounds according to Claim 1 in which n is 0.

4. (4-Chromanon-6-yl)acetic acid and its pharmaceutically acceptable salts.

5. (2,2-Dimethyl-4-chromanon-6-yl)acetic acid and its pharmaceutically acceptable salts.

6. 2-(8-Chloro-2,2-dimethyl-4-chromanon-6-yl)propionic acid and its pharmaceutically acceptable salts.

7. 2-(2,2-Dimethyl-4-chromanon-6-yl)propionic acid and its pharmaceutically acceptable salts.

8. 2-(8-Chloro-4-chromanon-6-yl)propionic acid and its pharmaceutically acceptable salts.

9. 2-(2,2-Dimethyl-2,3-dihydrobenzofuran-3-one-5-yl)propionic acid and its pharmaceutically acceptable salts.

10. An antiinflammatory, analgesic and antipyretic pharmaceutical composition containing at least one compound as claimed in Claim 1 as active ingredient, together with a carrier.

11. A compound as claimed in Claim 1 for use in treating symptoms with inflammation or pain.

European Patent Office

# EUROPEAN SEARCH REPORT

0087858
Application number

EP 83 30 0578

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | Chemical Abstracts vol. 92, no. 13, 31 March 1980 Columbus, Ohio, USA D.R. SHRIDHAR et al. "Synthesis and anti-inflammatory acitivity of some 2,3-dihydro-1,4-benzoxazepin-5(4H)-one-7-acetic acid esters" page 660, right-hand column, abstract no. 110976e & Indian Journal of Chemistry, Section B, vol. 17B, no. 2, 1979, pages 155-157 in connection with Chemical Abstracts, 10th Collective Index, vol. 86-95, 1977-1981, Columbus, Ohio, USA, Formulas, page 5182F, column 2, line 22, column 3, lines 55,57,58; Chemical Abstracs, 10th Collective Index, vol. 86-95, 1977-1981, Columbus, Ohio, USA Chemical Substances, page 9100CS, column 1, line 119, column 2, lines 103-105<br>--- | 1,2,4, 10,11 | C 07 D 311/22<br>C 07 D 307/83<br>A 61 K 31/35<br>A 61 K 31/34 |
| | | | **TECHNICAL FIELDS SEARCHED (Int. Cl. 3)** |
| X | US-A-4 151 179 (R.A. APPLETON et al.)<br>* Claims 1-15; examples 6-8, 10, 14, 16; column 5, line 8 - column 6, line 11 *<br>--- | 1,2,10 ,11 | A 61 K 31/34<br>A 61 K 31/35<br>C 07 D 307/00<br>C 07 D 311/04<br>C 07 D 311/22 |
| X | GB-A-1 499 323 (FISONS LTD.)<br>* Claims 19-34, 39, 41-44, 46, 49, 50 * & US - A - 4 057 641 (Cat. X)<br>--- -/- | 1,2,10 ,11 | |

The present search report has been drawn up for all claims

| Place of search | Date of completion of the search | Examiner |
|---|---|---|
| BERLIN | 26-04-1983 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

0087858

European Patent Office

# EUROPEAN SEARCH REPORT

Application number

EP 83 30 0578

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 077 066 (BENGER LABORATORIES LTD.) * Claims 1, 7; page 1, line 18 - page 2, line 10 * --- | 1,2 | |
| A | GB-A-2 014 566 (FARMITALIA C. ERBA S.P.A.) * Claims 1, 10 * ----- | 1,3,10 | |
| | | | TECHNICAL FIELDS SEARCHED (Int. Cl. 3) |

The present search report has been drawn up for all claims

| Place of search | Date of completion of the search | Examiner |
|---|---|---|
| BERLIN | 26-04-1983 | HASS C V F |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document